Europäisches Patentamt

European Patent Office

Office européen des brevets

(19)

(11) Veröffentlichungsnummer: **0 234 198 B1**

(12) **EUROPÄISCHE PATENTSCHRIFT**

(45) Veröffentlichungstag der Patentschrift:
05.09.90

(51) Int. Cl.⁵: **G01R 29/08, A61N 5/00**

(21) Anmeldenummer: 87100193.9

(22) Anmeldetag: 09.01.87

(54) Verfahren und Vorrichtung zur berührungslosen Bestimmung der Temperaturverteilung in einem Untersuchungsobjekt.

(30) Priorität: 23.01.86 DE 3601983

(43) Veröffentlichungstag der Anmeldung:
02.09.87 Patentblatt 87/36

(45) Bekanntmachung des Hinweises auf die Patenterteilung:
05.09.90 Patentblatt 90/36

(84) Benannte Vertragsstaaten:
DE FR GB IT NL

(56) Entgegenhaltungen:
EP-A- 0 217 111
GB-A- 2 000 335

L'ONDE ELECTRIQUE, Band 65, Nr. 1,
Januar/Februar 1985, Seiten 9-15, Paris, FR; Y. LEROY
et al.: "Applications de la radiométrie microonde en
génie biomédical"
ELECTRONICS LETTERS, Band 20, Nr. 9, 26. April 1984,
Seiten 380-382, London, GB; G. SCHALLER: "Inversion
of radiometric data from biological tissue by an
optimisation method"
IEEE Transactions on Microwave Theory and
Techniques,Vol.MTT-32,Nr.8,August 1984,S.829-835

(73) Patentinhaber: Siemens Aktiengesellschaft,
Wittelsbacherplatz 2, D-8000 München 2(DE)

(72) Erfinder: Klingenbeck, Klaus, Dr., Dannberger Weg 8,
D-8521 Hessdorf(DE)
Erfinder: Schittenheim, Rudolf, Dr.,
Von-Bezzel-Strasse 6, D-8520 Erlangen(DE)

**Beschreibung**

Die Erfindung betrifft ein Verfahren zur berührungslosen Bestimmung der Temperaturverteilung in einem inhomogenen Untersuchungsobjekt, wobei in einem ersten Verfahrensschritt mit einer Mikrowellenempfangseinrichtung die räumliche Verteilung der Amplitude und Phase der thermischen Eigenstrahlung des Untersuchungsobjekts ermittelt wird und wobei in einem zweiten Verfahrensschritt aus der räumlichen Verteilung der Amplituden und Phasen der thermischen Eigenstrahlung die räumliche Temperaturverteilung im Untersuchungobjekt abgeleitet wird. Die Erfindung betrifft weiterhin eine Vorrichtung zur Durchführung des Verfahrens.

Die Temperaturverteilung in einem Untersuchungsobjekt ist auf medizinischem Gebiet insbesondere dann von Interesse, wenn es sich bei dem Untersuchungsobjekt um einen menschlichen Patienten handelt. Dann nämlich besteht die Möglichkeit, den pathologischen Zustand eines Gebiets aufgrund der unterschiedlichen Wärmeausstrahlung im Vergleich zu gesundem Gewebe aus der Temperaturverteilung zu erkennen. In einem Hyperthermieverfahren kann eine lokale Aufheizung des von einem Krankheitsherd betroffenen Gebiets, z.B. eines Tumors, vorgenommen werden, um die Zersetzung krankhafter Zellen, insbesondere in Kombination mit Strahlentherapie, zu erreichen. Die Aufheizung darf einen kritischen Grenzwert nicht überschreiten und muß innerhalb des betroffenen Bereichs liegen, damit kein gesundes Gewebe geschädigt wird. Auch bei einem solchen Hyperthermieverfahren ist die Temperaturverteilung von Interesse zur genauen Lokalisierung des erwähnten Bereichs und damit zur Erfolgskontrolle der therapeutischen Maßnahmen.

Aufgrund der geringen Temperaturdifferenzen, die im menschlichen Körper auftreten, wird an ein Verfahren zur Ermittlung der Temperaturverteilung ein Anspruch auf hohe Genauigkeit gestellt. Dieser Anspruch ist besonders deswegen bei dielektrisch inhomogenen Untersuchungsobjekten nur sehr schwer zu erfüllen, weil bei der Messung der Eigenstrahlung neben der eigentlichen Temperatur auch die Verteilung der komplexen Dielektrizitätskonstante im Untersuchungskörper stark in das Meßergebnis eingeht.

Im menschlichen Körper beispielsweise variiert die Dielektrizitätskonstante zwischen einem Wert $\varepsilon = 10$ für Fett bis hin zu einem Wert $\varepsilon = 60$ für Knochen. Aufgrund der unterschiedlichen Dielektrizitätskonstanten wird die Eigenstrahlung, die von einem Punkt im Untersuchungskörper ausgeht, im Gewebe unterschiedlich gedämpft und gebrochen, so daß außerhalb des Untersuchungskörpers kein eindeutiges Ergebnis festgestellt werden kann. Bei gleicher Temperatur strahlt ein Bereich innerhalb des Untesuchungsobjekts mit hoher Dielektrizitätskonstante stärker als ein Bereich mit kleiner Dielektrizitätskonstante. Die Intensität der Eigenstrahlung außerhalb des Untersuchungsobjektes ist somit eine Funktion der Verteilung der Dielektrizitätskonstante und der Temperatur. Auch bei der Annahme, daß die Dielektrizitätskonstante z.B. einem Durchschnittswert entspricht, kann kein zufriedenstellendes, d.h. korrektes, Ergebnis erzielt werden. Es ist z.B. möglich, daß ein aufgrund der Eigenstrahlung zu 40°C gemessener erster Bereich im Untersuchungskörper im Vergleich zu einem zweiten Bereich, welcher mit 37°C gemessen wurde, in Wirklichkeit kälter ist als dieser zweite Bereich. Hat nämlich der erste Bereich, für den 40°C ermittelt wurde, eine sehr hohe Dielektrizitätskonstante, so ist seine Eigenstrahlung im Vergleich zu dem 37°C warmen Bereich, der voraussetzungsgemäß eine niedrige Dielektrizitätskonstante haben soll, verkehrt bewertet.

In dem Artikel "Aperture Synthesis Thermography" von Nicola C. Haslam et al., in IEEE Transactions on Microwave Theory and Techniques, Vol. MTT-32, Nr. 8, August 1984, S. 829–835, sind ein Verfahren und eine Vorrichtung der eingangs genannten Art beschrieben. Dort wird die thermische Eigenstrahlung eines Untersuchungsobjektes mit Hilfe einer Empfangsantenne empfangen. Das empfangene Signal wird in einem Computer nach Amplitude und Phase getrennt, die zur Berechnung der Temperaturverteilung dienen. Auf den komplexen Zusammenhang mit der Dielektrizitätskonstanten und die sich daraus ergebende Problematik bezüglich der Zuverlässigkeit der Meßergebnisse wrd in dieser Druckschrift nicht näher eingegangen; allerdings wird mittels Messungen an Eich-Dielektrika eine Eichung der verwendeten Vorrichtung durchgeführt, um unterschiedlich lange elektrische Weglängen oder unterschiedliche Dämpfungswerte im Untersuchungsobjekt berücksichtigen zu können.

Aufgabe der Erfindung ist es, ein Verfahren und eine Vorrichtung der eingangs genannten Art derart auszugestalten, daß die Temperaturverteilung im Untersuchungsobjekt mit großer Genauigkeit ermittelt werden kann.

Diese Aufgabe wird beim Verfahren der eingangs genannten Art erfindungsgemäß dadurch gelöst, daß vor dem zweiten Verfahrensschritt die am Untersuchungsobjekt herrschende räumliche Verteilung der komplexen Dielektrizitätskonstante gemessen wird, und daß im zweiten Verfahrensschritt bei der Ableitung der räumlichen Temperaturverteilung die räumliche Verteilung der komplexen Dielektrizitätskonstante berücksichtigt wird.

Auf diese Weise wird die Temperaturverteilung unter Berücksichtigung der inhomogenen Dämpfung und Streuung der thermischen Eigenstrahlung ermittelt.

Bezüglich der Vorrichtung wird die Aufgabe erfindungsgemäß durch eine Vorrichtung gelöst, bei der Untersuchungsobjekt zwischen einem Mikrowellensender und einer ersten Mikrowellenempfangseinrichtung angeordnet ist, wobei die erste Mikrowellenempfangseinrichtung mit einer Auswerteschaltung verbunden ist, die die räumliche Verteilung der Dielektrizitätskonstante im Untersuchungsobjekt ermittelt und dem ersten Eingang einer Recheneinrichtung zuführt, wobei zum Erfassen der thermischen Eigenstrahlung des Untersuchungsobjekts eine zweite Mikrowellenempfangseinrichtung vorgesehen ist, die die Meßwerte der thermischen Eigenstrahlung nach Betrag und Phase dem zweiten Eingang der Recheneinrichtung zuführt, und wobei am Ausgang der Recheneinrichtung ein die räumliche Temperaturverteilung im Untersuchungsobjekt darstellendes Signal abzugreifen ist.

Durch die Berücksichtigung der räumlichen Verteilung der Dielektrizitätskonstante im Untersuchungsobjekt ist sichergestellt, daß die von einem Bereich innerhalb des Untersuchungsobjekts emittierte Eigenstrahlung mit der ihr zukommenden Gewichtung verarbeitet wird. Aufgrund der gemessenen Verteilung der Dielektrizitätskonstante ist die Dämpfung und Streuung der Eigenstrahlung im homogenen Untersuchungsobjekt in die Berechnung mit einbezogen, so daß die außerhalb des Untersuchungsobjekts gemessene thermische Eigenstrahlung entsprechend ihrem individuellen Weg durch das Untersuchungsobjekt korrigiert wird. Die Korrektur wird dabei anhand der auf dem Ausbreitungsweg zwischen dem Objektbereich und der Empfangseinrichtung vorliegenden Verteilung der Dielektrizitätskonstante vorgenommen. Das Verfahren und die Vorrichtung ermöglichen es daher, selbst kleine Temperaturdifferenzen mit hoher räumlicher Auflösung in einem inhomogenen Untersuchungskörper darzustellen.

Eine besonders vorteilhafte Weiterbildung der Vorrichtung zeichnet sich dadurch aus, daß sie eine Erwärmungsquelle zur Aufheizung eines vorgegebenen Volumenbereiches im Untersuchungsobjekt umfaßt.

Ist das Untersuchungsobjekt ein Patient und der vorgegebene Volumenbereich ein krankhaftes Gebiet im Patienten, so kann die Erwärmungsquelle so gezielt eingesetzt werden, daß lediglich das krankhafte Gebiet, z.B. Geschwür, Krebszellen, überhitzt und eine hohe Abtötungsrate der defekten Zellen erzielt wird. Für eine wirkungsvolle Tumorbehandlung soll die Temperatur im Tumor und damit in etwa auch im umgebenden Normalgewebe möglichst nahe an die für das Normalgewebe zulässige Höchsttemperatur von 42 bis 43°C herangebracht werden. Während dieser Hyperthermie im vorgegebenen Volumenbereich ist eine Temperaturkontrolle mit einer Genauigkeit von Bruchteilen eines Grades Celsius und mit einer räumlichen Auflösung von wenigstens einem Zentimeter erforderlich. Eine solch genaue Temperaturkontrolle ist mit dem hier angegebenen Verfahren und der Vorrichtung zu erreichen.

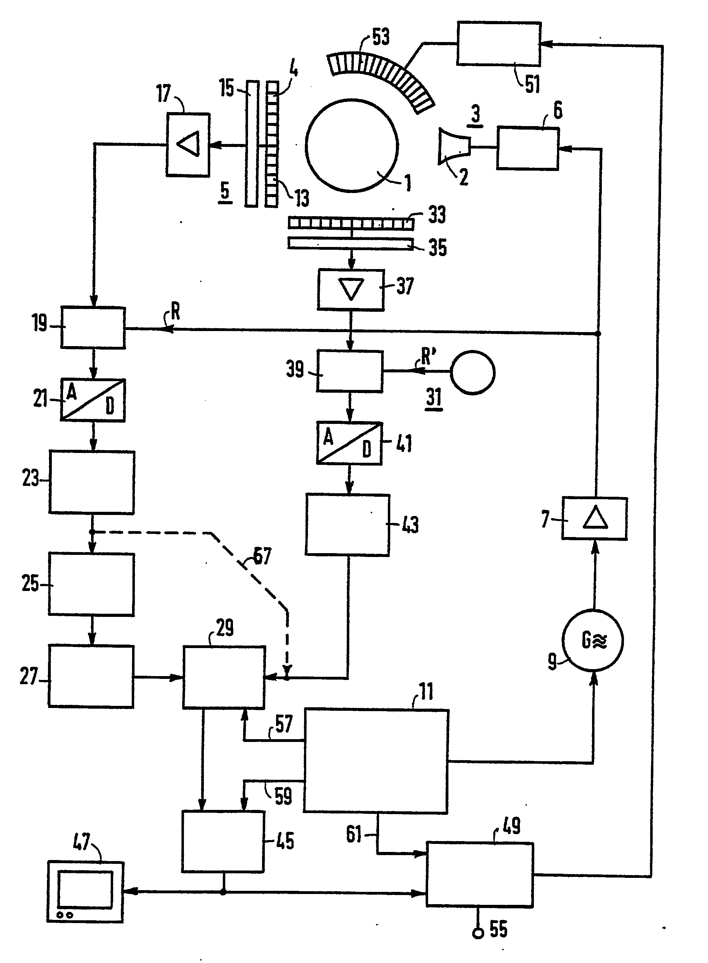

Weitere Vorteile und Ausgestaltungen der Erfindung ergeben sich aus der Beschreibung eines in der Figur dargestellten Ausführungsbeispiels in Verbindung mit den Unteransprüchen.

Die Figur zeigt den schematischen Aufbau einer Vorrichtung zur Aufnahme der Temperaturverteilung in einem Untersuchungsobjekt.

Ein Untersuchungsobjekt 1, insbesondere ein menschlicher Körper, ist zwischen dem Strahler 2 eines Mikrowellensenders 3 und der Empfangsantenne 4 einer ersten Mikrowellenempfangseinrichtung 5 angeordnet. Zur besseren Ankopplung ist der Zwischenraum mit einem Kopplungsmedium versehen. Die Sendefrequenz des Mikrowellensenders 3 läßt sich auf einen Wert im Bereich von 0,1 bis 10 GHz einstellen. Bevorzugt kommt ein Bereich zwischen 1 und 3 GHz infrage. Der Mikrowellensender 3 mit Strahler 2, Sendeeinheit 6, Sendeverstärker 7 und HF-Schwingungserzeuger oder HF-Oszillator 9 ist an eine Steuereinheit 11 angeschlossen. Der Sendeverstärker 7 ist so einstellbar, daß am Untersuchungsobjekt 1 eine Leistung von ca. 10 mW pro Quadratzentimeter zur Verfügung steht. Der HF-Schwingungserzeuger 9 arbeitet mit einer festen Frequenz, die einstellbar ist. Die eingestellte Frequenz entspricht derjenigen, die am Mikrowellensender 3 gewünscht ist. Von der Steuereinheit 11 erhält der HF-Schwingungserzeuger 9 des Mikrowellensenders 3 jeweils Start- und Stopbefehle. Der Einsatz des Mikrowellensenders 3 wird auf diese Weise von der Steuereinheit 11 gesteuert.

Die erste Mikrowellenempfangseinrichtung 5 umfaßt die Empfangsantenne 4, die insbesondere als zweidimensionales Array ausgebildet ist, einen nachgeschalteten ersten Multiplexer 15, einen ersten Empfangsverstärker 17, einen ersten phasenempfindlichen Detektor 19, einen ersten Analog-Digital-Wandler 21 und einen daran angeschlossenen ersten Speicher 23. Vom ersten Multiplexer 15 wird entsprechend einem Steuersignal ein vorgegebenes Empfangselement 13 der ersten Empfangsantenne 4 auf den ersten Empfangsverstärker 17 geschaltet. Das im ersten Verstärker 17 verstärkte Empfangssignal wird in dem ersten phasenempfindlichen Detektor 19 nach Amplitude und Phase zerlegt. Dazu wird dem ersten Detektor 19 ein Referenzsignal R zugeführt, welches vom Ausgang des Sendeverstärkers 7 abgegriffen ist. Die Amplitude und Phase werden in dem ersten Analog-Digital-Wandler 21 jeweils in ein digitales Signal gewandelt und in den ersten Speicher 23 ortsgetreu eingeschrieben.

Der Ausgang des ersten Speichers 23 ist mit einem Rechner 25 verbunden, der aus den gespeicherten Amplituden- und Phasendaten der empfangenen Mikrowellensignale die Verteilung der Dielektrizitätskonstante in dem Untersuchungsobjekt 1 errechnet. Diese dreidimensionale Verteilung wird in einem Zwischenspeicher 27 abgespeichert. Der Ausgang des Zwischenspeichers 27 ist mit dem ersten Eingang einer Recheneinrichtung 29 verbunden.

Von der Steuereinheit 11 wird der Mikrowellensender 3 durch die genannten Startbefehle aktiviert. Entsprechend der vorgewählten Sendefrequenz im Mikrowellenbereich wird das Untersuchungsobjekt 1 bestrahlt. Die Strahlung wird im Untersuchungsobjekt 1 gedämpft und gestreut; die durchgelassene Strahlung wird von der ersten Mikrowellenempfangseinrichtung 5 detektiert. Die nach Betrag und Phase zerlegten Empfangssignale der Mikrowellenempfangseinrichtung 5 werden einer Auswerteschaltung, die aus dem Rechner 25 und dem Zwischenspeicher 27 besteht, zugeführt. In der Auswerteschaltung 25, 27 wird aus den empfangenen Mikrowellensignalen die Verteilung der Dielektrizitätskonstante im Untersuchungsobjekt 1 bestimmt.

Ein Verfahren und eine Vorrichtung zur Ermittlung der räumlichen Verteilung der Dielektrizitätskonstante sind in der EP-A 0 217 111 offenbart. Das Verfahren kann für vorliegendes Verfahren übernommen werden.

Auf das Untersuchungsobjekt 1 ist eine zweite Mikrowellenempfangseinrichtung 31 gerichtet, die aus einer zweiten Empfangsantenne 33 in Form eines zweidimensionalen Arrays, einem zweiten Multiplexer 35, einem zweiten Empfangsverstärker 37, einem zweiten phasenempfindlichen Detektor 39, der von einem Referenzsignal R' beaufschlagt ist, einem zweiten Analog-Digital-Wandler 41 und einem zweiten Speicher 43 besteht. Der Ausgang des zweiten Analog-Digital-Wandlers 41 ist mit dem zweiten Speicher 43 verbunden, dessen Ausgang an den zweiten Eingang der Recheneinrichtung 29 geführt ist. Die Arbeitsweise der zweiten Empfangseinrichtung 31 ist analog zu derjenigen der ersten Empfangseinrichtung 5. Am Ausgang des zweiten Speichers 43 stehen also, unter Zuordnung zum Empfangsort, die empfangenen Mikrowellensignale nach Amplitude und Phase zerlegt zur Verfügung. Die Empfangsantenne 33 ist dabei zweckmäßigerweise rechtwinklig zur Abstrahlrichtung des Strahlers 2 aufgestellt.

Mit Hilfe des Mikrowellensenders 3 und der ihm zugeordneten ersten Empfangseinrichtung 5 wird die Verteilung der elektrischen Dielektrizitätskonstante im Untersuchungsobjekt 1 gemäß der genannten EP-A 0 217 111 ermittelt. Wenn die Temperaturverteilung im Untersuchungsobjekt 1 nur sehr kleine Unterschiede aufweist und zeitlich konstant ist, wie es bei einem Patienten der Fall sein kann, bleibt die Verteilung der Dielektrizitätskonstante $\varepsilon$ über den gesamten Untersuchungszeitraum hinweg praktisch unverändert. In einem solchen Fall wird die Eigenstrahlung über die zweite Mikrowellenempfangseinrichtung 31 zweckmäßigerweise bei ausgeschaltetem Mikrowellensender 3 gemessen, da eine erneute Aufnahme der Verteilung von $\varepsilon$ nicht nötig ist. Die erste und zweite Empfangseinrichtung 5, 31 brauchen nicht gleichzeitig betrieben zu werden, und die Empfangsverstärker 17, 37 können auf dieselbe Frequenz von beispielsweise 3 GHz abgestimmt sein.

Erfolgt die Untersuchung in Zusammenhang mit einer Hyperthermiebehandlung, also einer Erwärmung innerhalb des Untersuchungsobjektes 1 um eine vorgegebene Temperatur, und ist eine Veränderung in der Verteilung der Dielektrizitätskonstante $\varepsilon$ im Untersuchungsobjekt 1 zu erwarten, so muß die Messung der Verteilung der Dielektrizitätskonstante $\varepsilon$ während der Untersuchungsdauer wiederholt vorgenommen werden. Soll dieses im Parallelbetrieb mit der Ermittlung der Eigenstrahlung des Untersuchungsobjekts 1 geschehen, so werden der erste und zweite Empfangsverstärker 17, 37 zweckmäßigerweise auf unterschiedliche Frequenzen abgestimmt. Der erste Empfangsverstärker 17 ist dann auf die Sendefrequenz des Mikrowellensenders 3 von z.B. 3 GHz und der zweite Empfangsverstärker 37 auf eine Frequenz von beispielsweise 1 GHz abgestimmt. Auf diese Weise läßt sich ausschließen, daß die in das Untersuchungsobjekt 1 eingestrahlte Mikrowellenstrahlung das Ergebnis der Ermittlung der Eigenstrahlung des Untersuchungsobjektes 1 verfälscht. Das Referenzsignal R' des phasenempfindlichen Detektors 39 ist dann ein festes Referenzsignal der Frequenz 1 GHz.

Die Recheneinrichtung 29 weist einen Ausgang auf, an dem nach dem Rechenvorgang die räumliche Temperaturverteilung im Untersuchungsobjekt 1 zu Verfügung steht. Auf den Algorithmus, der von der Recheneinrichtung 29 verwendet wird, wird später näher eingegangen. Der Ausgang der Recheneinrichtung 29 ist zweckmäßigerweise mit einem weiteren Speicher 45 zur Speicherung der räumlichen Temperaturverteilung verbunden. Der Ausgang des Speichers 45 ist an ein Anzeigegerät 47 angeschlossen. Dieses ist beispielsweise ein Bildschirm, auf welchem die Temperaturverteilung von gewünschten Ebenen im Untersuchungsobjekt 1 darstellbar ist. Der Ausgang des weiteren Speichers 45 ist außerdem mit einer Steuereinrichtung 49 verbunden, welche eine Erwärmungsquelle 51 mit Abstrahleinrichtung 53 ansteuert. Die Abstrahleinrichtung 53 der Erwärmungsquelle 51 ist auf das Untersuchungsobjekt 1 ausgerichtet und kann dort einen vorgegebenen Volumenbereich aufheizen.

Von der Steuereinrichtung 49 wird das Temperaturmaximum in der räumlichen Temperaturverteilung im Untersuchungsobjekt 1 ermittelt. Überschreitet das Temperaturmaximum einen vorgegebenen Grenzwert, der bei einem Patienten als Untersuchungsobjekt 1 z.B. bei etwa 43°C liegt, so wird die Erwärmungsquelle 51 von der Steuereinrichtung 49 abgeschaltet.

Weiterhin werden in der Steuereinrichtung 49 die zu dem Temperaturmaximum gehörigen Koordinaten im Untersuchungsobjekt 1 ermittelt. Diese Koordinaten werden mit vorgegebenen Koordinaten eines Volumenbereiches im Untersuchungsobjekt 1 verglichen, die über einen Eingang 55 in die Steuereinrichtung 49 eingegeben werden. Die vorgegebenen Koordinaten sind beispielsweise mit Hilfe einer Computertomographie- oder NMR-Aufnahme ermittelt worden und sind die Koordinaten des Zentrums eines Tumors. In Abhängigkeit von einem Vergleich der vorgegebenen Koordinaten mit denen des ermittelten Temperaturmaximums wird von der Steuereinrichtung 49 die Abstrahlrichtung der Abstrahleinrichtung 53 automatisch so geführt, daß die Koordinaten des Maximums der Temperaturverteilung mit den vorgegebenen Koordinaten, also mit dem Tumor, zusammenfallen. Auf diese Weise ist sichergestellt, daß bei einem Hyperthermieverfahren das Temperaturmaximum in dem gewünschten Bereich des Untersuchungsobjekts 1 liegt und der Tumor durch Wärme zerstört wird.

Zur Ansteuerung der verschiedenen Bauelemente, z.B. des Rechners 29, des Speichers 45 und der Steuereinrichtung 49, sind von der Steuereinheit 11 ausgehende Steuerleitungen 57, 59 bzw. 61 vorgesehen.

Die erste und zweite Mikrowellenempfangseinrichtung 5, 31 können nach einer Ausgestaltung zu einer gemeinsamen Empfangseinrichtung zusammengefaßt sein. Von der Empfangsantenne 4 bis hin zu dem Speicher 23 sind dann z.B. lediglich die Baukomponenten der Empfangseinrichtung 5 vorgesehen. Die Empfangseinrichtung 5 dient dann sowohl zur Ermittlung der Verteilung der Dielektrizitätskonstante $\varepsilon$ als auch zur Ermittlung der Eigenstrahlung des Untersuchungsobjekts 1. Bei dieser Ausgestaltung ist Voraussetzung, daß der Mikrowellensender 3 abgeschaltet ist, wenn die Eigenstrahlung des Untersuchungsobjekts 1 ermittelt wird. Eine solche Verriegelung der Arbeitsweise wird in der Steuereinheit 11 vorgenommen. Im Falle der gemeinsamen Mikrowellenempfangseinrichtung ist vom ersten Speicher 23 eine Verbindung 67 (gestrichelt gezeichnet) zu dem zweiten Eingang der Recheneinrichtung 29 gelegt. Über diese Verbindung 67 werden Amplitude und Phase der ermittelten thermischen Eigenstrahlung der Recheneinrichtung 29 zugeführt.

In dieser Ausgestaltung ist also lediglich der sogenannte "Off-Line"-Betrieb möglich. D.h. die Ermittlung der Temperaturverteilung aus der Dielektrizitätskonstanten und die der thermischen Eigenstrahlung erfolgen nacheinander und nicht gleichzeitig.

Bei Vorhandensein von zwei Mikrowellenempfangseinrichtungen 5, 31 dagegen ist ein sogenannter "On-Line"-Betrieb möglich. Wie bereits erwähnt, sind die beiden Empfangsverstärker 17, 37 für diesen Fall auf unterschiedliche Frequenzen von z.B. 3 bzw. 1 GHz abgestimmt. Der Zwischenspeicher 27 kann in diesem Fall weggelassen werden. Der Ausgang des Rechners 25 ist dann direkt mit dem ersten Eingang der Recheneinrichtung 29 verbunden. Diese erhält an ihren beiden Eingängen jeweils für einen Raumpunkt in dem Untersuchungsobjekt 1 sowohl den Wert der Dielektrizitätskonstante $\varepsilon$ als auch den der Amplitude und Phase der thermischen Eigenstrahlung. Aus dieser Information kann die Recheneinrichtung 29 die zugehörige Temperatur T bestimmen.

In der Recheneinrichtung 29 wird folgende Integralgleichung aufgelöst:

$$S(\underline{r}) \propto \int d^3 r' \; \mathcal{E}(\underline{r}') \; T(\underline{r}') \; G(\underline{r} - \underline{r}') \qquad (1)$$

Darin bedeuten $S(\underline{r})$ die thermische Eigenstrahlung des Untersuchungsobjekts 1 am Ort $\underline{r}$. Diese Eigenstrahlung $S(\underline{r})$ ist anhand der Amplitude und Phase der auf der Empfangsantenne 33 eintreffenden Mikrowellenstrahlung bekannt. $\varepsilon(\underline{r}')$ ist die Verteilung der Dielektrizitätskonstante im Untersuchungsobjekt 1. Diese wird z.B. gemäß der erwähnten EP-A 0 217 111 ermittelt und dem ersten Eingang der Recheneinrichtung 29 entweder aus dem weiteren Speicher 27 heraus oder direkt vom Rechner 25 her eingegeben. $T(\underline{r})$ ist die gewünschte Temperaturverteilung, nach welcher die obige Integralgleichung (1) aufzulösen ist. $G(\underline{r}-\underline{r}')$ ist die Green'sche Funktion, die die Ausbreitung der Mikrowellenstrahlung vom Ort $\underline{r}'$ eines Punktes im Untersuchungsobjekt 1 zum Ort $\underline{r}$ eines Punktes in der Empfangsantenne 33 beschreibt. $\underline{r}$ und $\underline{r}'$ sind also Ortsvektoren von einem Ursprungsort zur Empfangsantenne 33 bzw. zum Untersuchungsobjekt 1.

Die Green'sche Funktion $G(\underline{r})$ ist durch eine Integralgleichung (2) definiert:

$$G(\underline{r}) = G_0(\underline{r}) + \int d^3 r' \; G_0(\underline{r}-\underline{r}') \; (k_0^2 - k_0^2(\underline{r}')) \; G(\underline{r}'), \qquad (2)$$

wobei $G_0$ die Green'sche Funktion für die Ausbreitung der Mikrowellenstrahlung im umgebenden Koppelmedium ist. $G_0$ ist definiert durch folgende Differentialgleichung (3):

$$(\Delta + k_0^2) \; G_0(\underline{r}-\underline{r}') = \delta(\underline{r}-\underline{r}') \qquad (3)$$

Hierbei bezeichnet ko die Wellenzahl der Mikrowellenstrahlung im Koppelmedium und im Untersuchungsobjekt 1. Es gilt

$$k_0 = \frac{\omega}{c} \cdot \sqrt{\varepsilon_0 \mu_0} \qquad \text{und} \qquad k(\underline{r}) = \frac{\omega}{c} \cdot \sqrt{\varepsilon(\underline{r}) \mu(\underline{r})}$$

wobei $\varepsilon_0$ und $\mu_0$ die Dielektrizitätskonstante bzw. Permeabilität des Koppelmediums ist, und wobei $\varepsilon(\underline{r})$ und $\mu(\underline{r})$ die räumliche Verteilung der entsprechenden Größen im Untersuchungsobjekt 1 bezeichnen. $\Delta$ ist der Laplace'sche Differentialoperator

$$\Delta = \frac{\partial^2}{\partial x^2} + \frac{\partial^2}{\partial y^2} + \frac{\partial^2}{\partial z^2}$$

der auf die Ortskoordinate $\underline{r}$ wirkt, und $\delta(\underline{r}-\underline{r})$ ist die Dirac'sche $\delta$-Funktion. Die analytische Form von $G_0$ sind die bekannten Kugelwellen

$$G_0(\underline{r}-\underline{r}') = \frac{1}{\sqrt{4\pi}} \cdot \frac{e^{ik_0|\underline{r}-\underline{r}'|}}{|\underline{r}-\underline{r}'|} \tag{4}$$

$\omega$ und $c$ sind die Kreisfrequenz der Mikrowellenstrahlung bzw. die Lichtgeschwindigkeit. Wenn also $\epsilon(\underline{r})$ bzw. $\epsilon(\underline{r}) \cdot \mu(\underline{r})$ aus der Mikrowellenabbildung bekannt sind, kann die benötigte Green'sche Funktion G aus obiger Integralgleichung (2) berechnet werden. Da die Integralgleichung (2) für G die inhomogene Verteilung der dielektrischen Eigenschaften des Untersuchungsobjekts enthält, beschreibt $G(\underline{r})$ die Ausbreitung der Strahlung von einem Punkt im Untersuchungsobjekt zum Detektor unter Berücksichtigung der Dämpfung und Streuung im Untersuchungsobjekt.

Die Auflösung der Integralgleichung (1) erfolgt über eine Transformation in den Fourierraum, so daß folgende Beziehung gilt: (* bedeutet Durchführung der Fourierfaltung)

$$(\mathcal{E} * T)(\underline{k}) \cdot G(\underline{k}) \propto S(\underline{k}) \tag{5}$$

$$(\mathcal{E} * T)(\underline{k}) \propto \frac{S(k)}{G(k)} = R(\underline{k}) \tag{6}$$

$\underline{k}$ bedeutet hier den dreidimensionalen Ortsfrequenzvektor. Nach Rücktransformation in den Ortsraum durch inverse Fouriertransformation läßt sich die Gleichung (6) umschreiben zu:

$$\mathcal{E}(\underline{r}) \cdot T(\underline{r}) = FT^{-1}\left[(S/G)\right] = R(\underline{r}) \tag{7}$$

Hier bedeutet $FT^{-1}$ die inverse Fourier-Transformation. Daraus läßt sich $T(\underline{r})$ nach der folgenden Gleichung (8) ermitteln:

$$T(\underline{r}) = \frac{1}{\mathcal{E}(r)} \ R(\underline{r}) \tag{8}$$

Das Verfahren zur Bestimmung der Temperaturverteilung $T(\underline{r})$ im Untersuchungsobjekt 1 läuft folgendermaßen ab. Der Mikrowellensender 3 wird aktiviert und gibt Mikrowellenstrahlen in Richtung auf das Untersuchungsobjekt 1 ab. Sie werden im inhomogenen Untersuchungsobjekt 1 räumlich unterschiedlich stark gedämpft und gestreut. Die aus dem Untersuchungsobjekt 1 wieder austretende Mikrowellenstrahlung wird von der ersten Empfangseinrichtung 5 erfaßt und nach Amplitude und Phase zerlegt. In dem Rechner 25 wird die Verteilung der Dielektrizitätskonstante $\epsilon(\underline{r})$ errechnet und für jeden Raumpunkt im Untersuchungsobjekt 1 in den Zwischenspeicher 27 eingeschrieben. Im Zwischenspeicher 27 steht anschließend die räumliche Verteilung der Dielektrizitätskonstante $\epsilon(\underline{r})$ im Untersuchungsobjekt 1 zur Verfügung.

Daraufhin wird der Mikrowellensender 3 abgeschaltet. Nun wird über die zweite Mikrowellenempfangseinrichtung 31 die thermische Eigenstrahlung des Untersuchungsobjekts 1 ermittelt. In der Recheneinrichtung 29 wird entsprechend dem vorher angegebenen Algorithmus aus der Verteilung der Dielektrizitätskonstante und der ermittelten Eigenstrahlung die Temperaturverteilung im Untersuchungsobjekt 1 berechnet und in den Speicher 45 eingeschrieben.

Im Falle eines Patienten als Untersuchungsobjekt 1, bei welchem ein Tumor durch thermische Zersetzung zerstört werden soll, schließt sich eine Erwärmung in dem vorgegebenen Volumenbereich des Untersuchungsobjekts 1 an. Dazu wird die Erwärmungsquelle 51 für einen gewissen Zeitraum angeschaltet. Daraufhin wird erneut über die zweite Mikrowellenempfangseinrichtung 31 die Eigenstrahlung im Untersuchungsobjekt 1 aufgenommen und mit der im Zwischenspeicher 27 noch vorhandenen Verteilung der Dielektrizitätskonstante $\epsilon(\underline{r})$ zusammen zu einer räumlichen Temperaturverteilung verarbeitet. Die aufgrund der Wärmebehandlung erzielte Aufheizung im Untersuchungsobjekt 1 ist aus der neuen Temperaturverteilung ersichtlich.

Alternativ hierzu ist es möglich, erst wieder den Mikrowellensender 3 zu aktivieren und über die erste Empfangseinrichtung 5 eine neue Verteilung der Dielektrizitätskonstante $\epsilon(\underline{r})$ aufzunehmen. Anschlie-

ßend daran wird die Eigenstrahlung ermittelt und die neue Temperaturverteilung errechnet. Dieser Vorgang kann wiederholt werden, bis die kritische Temperatur von ca. 43°C für das Gewebe erreicht ist. Bei diesem Anwendungsfall läßt sich das Verfahren zur Temperaturbestimmung zur Kontrolle der Hyperthermiebehandlung einsetzen. Die Frequenz der Erwärmungsquelle 51 sollte dabei so gewählt sein, daß sie außerhalb der Bandbreite der Frequenzen der ersten und zweiten Empfangseinrichtung 5, 31 liegt.

## Patentansprüche

1. Verfahren zur berührungslosen Bestimmung der Temperaturverteilung in einem inhomogenen Untersuchungsobjekt (1), wobei in einem ersten Verfahrensschritt mit einer Mikrowellenempfangseinrichtung (31) die räumliche Verteilung der Amplitude und Phase der thermischen Eigenstrahlung des Untersuchungsobjekts (1) ermittelt wird und wobei in einem zweiten Verfahrensschritt aus der räumlichen Verteilung der Amplituden und Phasen der thermischen Eigenstrahlung die räumliche Temperaturverteilung im Untersuchungsobjekt (1) abgeleitet wird, dadurch gekennzeichnet, daß vor dem zweiten Verfahrensschritt die am Untersuchungsobjekt (1) herrschende räumliche Verteilung der komplexen Dielektrizitätskonstante gemessen wird, und daß im zweiten Verfahrensschritt bei der Ableitung der räumlichen Temperaturverteilung die räumliche Verteilung der komplexen Dielektrizitätskonstante berücksichtigt wird.

2. Verfahren nach Anspruch 1, dadurch gekennzeichnet, daß die Temperaturverteilung ($T(\underline{r})$) durch Auflösen einer Integralgleichung, welche sowohl die räumliche Verteilung der Amplituden und Phasen der Eigenstrahlung $S(\underline{r})$ als auch die räumliche Verteilung der komplexen Dielektrizitätskonstanten umfaßt, abgeleitet wird.

3. Verfahren nach Anspruch 2, dadurch gekennzeichnet, daß die Integralgleichung vom Typ

$$S(\underline{r}) \;\propto\; \int d^3 r' \; \mathcal{E}(\underline{r}') \; T(\underline{r}') \quad (\underline{r} - \underline{r}')$$

ist, wobei $S(\underline{r})$ die thermische Eigenstrahlung des Untersuchungsobjekts (1) am Ort $\underline{r}$, $\varepsilon(\underline{r}')$ die Verteilung der Dielektrizitätskonstante im Untersuchungsobjekt (1), $T(\underline{r}')$ die Temperaturverteilung im Untersuchungsobjekt (1), nach der die Integralgleichung aufzulösen ist, und $G(\underline{r}-\underline{r}')$ die Green'sche Funktion bedeuten.

4. Hyperthermieverfahren, bei welchem ein vorgegebener Volumenbereich innerhalb des Untersuchungsobjektes (1) erwärmt wird und die Bestimmung der Temperaturverteilung innerhalb des Untersuchungsobjektes (1) nach einem Verfahren nach einem der Ansprüche 1 bis 3 erfolgt.

5. Hyperthermieverfahren nach Anspruch 4, dadurch gekennzeichnet, daß die Erwärmung des vorgegebenen Volumenbereiches innerhalb des Untersuchungsobjektes (1) vor der berührungslosen Bestimmung der Temperaturverteilung erfolgt.

6. Hyperthermieverfahren nach Anspruch 4 oder 5, dadurch gekennzeichnet, daß der Volumenbereich auf eine Temperatur etwa zwischen 42 und 43°C erwärmt wird.

7. Vorrichtung zur Durchführung des Verfahrens nach einem der Ansprüche 1 bis 3, bei der das Untersuchungsobjekt (1) zwischen einem Mikrowellensender (3) und einer ersten Mikrowellenempfangseinrichtung (5) angeordnet ist, wobei die erste Mikrowellenempfangseinrichtung (5) mit einer Auswerteschaltung (25, 27) verbunden ist, die die räumliche Verteilung der Dielektrizitätskonstante im Untersuchungsobjekt (1) ermittelt und dem ersten Eingang einer Recheneinrichtung (29) zuführt, wobei zum Erfassen der thermischen Eigenstrahlung des Untersuchungsobjekts (1) eine zweite Mikrowellenempfangseinrichtung (31) vorgesehen ist, die die Meßwerte der thermischen Eigenstrahlung nach Betrag und Phase dem zweiten Eingang der Recheneinrichtung (29) zuführt, und wobei am Ausgang der Recheneinrichtung (29) ein die räumliche Temperaturverteilung im Untersuchungsobjekt (1) darstellendes Signal abzugreifen ist.

8. Vorrichtung nach Anspruch 7, dadurch gekennzeichnet, daß die erste und die zweite Mikrowellenempfangseinrichtung (5, 31) auf unterschiedliche Frequenzen abgestimmt sind.

9. Vorrichtung nach Anspruch 7 oder 8, dadurch gekennzeichnet, daß die erste und zweite Mikrowellenempfangseinrichtung (5, 31) auf eine bzw. je eine Frequenz zwischen 0,1 und 10 GHz abgestimmt sind.

10. Vorrichtung nach Anspruch 7, dadurch gekennzeichnet, daß die erste und die zweite Mikrowellenempfangseinrichtung (5, 31) zu einer gemeinsamen Mikrowellenempfangseinrichtung zusammengefaßt sind, welche sowohl mit der Auswerteschaltung (25, 27) zur Ermittlung der räumlichen Verteilung der Dielektrizitätskonstante als auch mit dem zweiten Eingang der Recheneinrichtung (29) verbunden ist.

11. Vorrichtung nach einem der Ansprüche 7 bis 10, dadurch gekennzeichnet, daß die Auswerteschaltung (25, 27) zur Ermittlung der räumlichen Verteilung der Dielektrizitätskonstante einen Speicher (27) umfaßt, in dem die Dielektrizitätskonstante einschreibbar ist, und daß der Ausgang des Speichers (27) mit der Recheneinrichtung (29) verbunden ist.

12. Vorrichtung nach einem der Ansprüche 7 bis 11, dadurch gekennzeichnet, daß sie eine Erwärmungsquelle (51) zur Aufheizung eines vorgegebenen Volumenbereiches im Untersuchungsobjekt (1) umfaßt.

13. Vorrichtung nach Anspruch 12, dadurch gekennzeichnet, daß eine Steuereinrichtung (49) vorgesehen ist, die die Erwärmungsquelle (51) abschaltet, wenn das von der Recheneinrichtung (29) ermittelte Maximum der Temperaturverteilung einen vorgegebenen Grenzwert übersteigt.

14. Vorrichtung nach Anspruch 13, dadurch gekennzeichnet, daß der vorgegebene Grenzwert etwa 43°C beträgt.

15. Vorrichtung nach einem der Ansprüche 10 bis 14, dadurch gekennzeichnet, daß eine Steuereinheit (11) vorgesehen ist, die den Mikrowellensender (3) abschaltet, wenn die gemeinsame Mikrowellenempfangseinrichtung (5) die thermische Eigenstrahlung des Untersuchungsobjekts (1) erfaßt.

16. Vorrichtung nach einem der Ansprüche 11 bis 15, dadurch gekennzeichnet, daß eine Steuereinheit (11) vorgesehen ist, die den Mikrowellensender (3) zuschaltet, wenn die von der Auswerteschaltung (25, 27) ermittelte räumliche Verteilung der Dielektrizitätskonstante in den Speicher (27) eingeschrieben werden soll.

17. Vorrichtung nach einem der Ansprüche 12 bis 16, dadurch gekennzeichnet, daß die Erwärmungsquelle (51) mit einer Steuereinrichtung (49) verbunden ist, deren einem Eingang die Temperaturverteilung und deren anderem Eingang (53) die Koordinaten des vorgegebenen Volumenbereiches im Untersuchungsobjekt (1) zugeführt sind, daß in der Steuereinrichtung (49) aus der Temperaturverteilung die Koordinaten des Temperaturmaximums ermittelt und mit den vorgegebenen Koordinaten verglichen werden, und daß die Abstrahlrichtung der Erwärmungsquelle (51) automatisch so geführt wird, daß bei einer erneuten Erwärmung die Koordinaten des Maximums der Temperaturverteilung mit den vorgegebenen Koordinaten zusammenfallen.

18. Vorrichtung nach einem der Ansprüche 12 bis 17, dadurch gekennzeichnet, daß die Erwärmungsquelle (51) eine Strahlungsquelle mit einer Sendefrequenz ist, die außerhalb der Bandbreite der Frequenz der Empfangseinrichtung (5, 31) liegt.

19. Vorrichtung nach einem der Ansprüche 10 bis 18, dadurch gekennzeichnet, daß die aktive Messung der Dielektrizitätskonstante und die passive Messung der thermischen Eigenemission alternierend erfolgen.

## Claims

1. A process for non-contact determination of the temperature distribution in an inhomogeneous object (1) to be examined, wherein in a first step the three-dimensional amplitude and phase distribution of the characteristic thermal radiation of the examination object (1) is detected by means of a microwave receiver (31), and in a second step the three-dimensional temperature distribution in the examination object (1) is derived from the three-dimensional amplitude and phase distribution of the characteristic thermal radiation, characterised in that before the second step the three-dimensional distribution of the complex dielectric constant prevailing in the examination object (1) are measured, and that in the second step, when deriving the three-dimensional temperature distribution, the three-dimensional distribution of the complex dielectric constant is taken into account.

2. A process according to claim 1, characterised in that the temperature distribution ($T(\underline{r})$) is derived by solving an integral equation which expresses both the three-dimensional amplitude and phase distribution of the characteristic radiation $S(\underline{r})$ nd the three-dimensional distribution of the complex dielectric constant.

3. A process according to claim 2, characterised in that the integral equation is of the type:

$$S(\underline{r}) \propto \int d^3 r' \, \mathcal{E}(\underline{r}') \, T(\underline{r}') \quad (\underline{r} - \underline{r}')$$

where $S(\underline{r})$ is the characteristic thermal radiation of the examination object (1) at a location $\underline{r}$, $\varepsilon(\underline{r})$ is the distribution of the dielectric constant in the examination object (1), $T(\underline{r})$ is the temperature distribution in the examination object (1) according to which the integral equation is to be solved, and $G(\underline{r}-\underline{r}')$ is Green's function.

4. A hyperthermic process wherein a preselected volume region within the examination object (1) is heated, and the temperature distribution within the examination object (1) is determined by a process according to any one of claims 1 to 3.

5. A hyperthermic process according to claim 4, characterised in that heating of the preselected volume region within the examination object (1) is performed before the non-contact determination of the temperature distribution.

6. A hyperthermic process according to claim 4 or claim 5, characterised in that the volume region is heated to a temperature of about between 42 and 43°C.

7. An apparatus for carrying out the process according to any one of claims 1 to 3, wherein the examination object (1) is arranged between a microwave transmitter (3) and a first microwave receiver (5), the first microwave receiver (5) being connected to an analysis circuit (25, 27) which determines the three-dimensional distribution of the dielectric constant in the examination object (1) and supplies this to the first input of a computer (29), and to detect the characteristic thermal radiation of the examination object (1) a second microwave receiver (31) is provided which supplies the measured values of the characteristic thermal radiation according to amount and phase to the second input of the computer (29), and a signal representing the three-dimensional temperature distribution in the examination object (1) can be taken off at the output of the computer (29).

8. An apparatus according to claim 7, characterised in that the first and the second microwave receiver (5, 31) are tuned to different frequencies.

9. An apparatus according to claim 7 or claim 8, characterised in that the first and the second microwave receiver (5, 31) or each of them is or are tuned to a frequency between 0.1 and 10 GHz.

10. An apparatus according to claim 7, characterised in that the first and the second microwave receiver (5, 31) are combined to form a common microwave receiver which is connected both to the analysis circuit (25, 27) for determining the three-dimensional distribution of the dielectric constant and to the second input of the computer (29).

11. An apparatus according to any one of claims 7 to 10, characterised in that the analysis circuit (25, 27) for determining the three-dimensional distribution of the dielectric constant includes a store (27) into which the dielectric constant can be written, and that the output of the store (27) is connected to the computer (29).

12. An apparatus according to any one of claims 7 to 11, characterised in that it includes a heating source (51) for heating up a preselected volume region in the examination object (1).

13. An apparatus according to claim 12, characterised in that a control device (49) is provided wich switches the heating source (51) off when the maximum of the temperature distribution determined by the computer (29) exceeds a preselected threshold value.

14. An apparatus according to claim 13, characterised in that the preselected threshold value is about 43°C.

15. An apparatus according to any one of claims 10 to 14, characterised in that a control unit (11) is provided which switches the microwave transmitter (3) off when the common microwawe receiver (5) has detected the characteristic thermal radiation of the examination object (1).

16. An apparatus according to any one of claims 11 to 15, characterised in that a control unit (11) is provided which switches the microwave transmitter (3) on when the three-dimensional distribution of the dielectric constant determined by the analysis circuit (25, 27) is to be written into the store (27).

17. An apparatus according to any one of claims 12 to 16, characterised in that the heating source (51) is connected to a control device (49) of which one input is supplied with the temperature distribution and the other input (53) with the coordinates of the preselected volume region in the examination object (1), and that in the control device (49) the coordinates of the temperature maximum are derived from the temperature distribution and compared with the preselected coordinates, and that the direction of radiation of the heating source (51) is automatically made such that on renewed heating the coordinates of the maximum of the temperature distribution coincide with the preselected coordinates.

18. An apparatus according to any one of claims 12 to 17, characterised in that the heating source (51) comprises a radiation source with a transmission frequency lying outside the band width of the frequency of the receiver (5, 31).

19. An apparatus according to any one of claims 10 to 18, characterised in that the active measurement of the dielectric constant and the passive measurement of the characteristic thermal emission take place alternately.

## Revendications

1. Procédé pour déterminer la répartition de la température dans un objet inhomogène à examiner (1), du type dans lequel on détermine, dans une première phase opératoire et à l'aide d'un dispositif récepteur hyperfréquence (31), la répartition spatiale de l'amplitude et de la phase du rayonnement thermique propre de l'objet à examiner (1), et dans lequel, dans une seconde phase opératoire, on détermine, à partir de la répartition spatiale des amplitudes et des phases du rayonnement thermique propre, la répartition spatiale de la température dans l'objet à examiner (1), caractérisé par le fait qu'avant la seconde phase opératoire, on mesure la répartition spatiale, dans l'objet à examiner (1), de la constante diélectrique complexe, et que dans la seconde phase opératoire, on tient compte, lorsqu'on dérive la répartition spatiale de la température, de la répartition spatiale de la constante diélectrique complexe.

2. Procédé selon la revendication 1, caractérisé par le fait que la répartition de la température ($T(\underline{r})$) est dérivée par la résolution d'une équation intégrale qui comprend aussi bien la répartition spatiale des amplitudes et des phases du rayonnement propre $S(\underline{r})$ que la répartition spatiale des constantes diélectriques complexes.

3. Procédé selon la revendication 2, caractérisé par le fait que l'équation intégrale est du type

$$S(\underline{r}) \propto \int d^3 r' \; \mathcal{E}(\underline{r}') \; T(\underline{r}') \quad (\underline{r} - \underline{r}') \quad ,$$

dans laquelle $S(\underline{r})$ représente la radiation thermique propre de l'objet à examiner (1) à l'emplacement $\underline{r}$,

$\varepsilon(\underline{r})$ représente la répartition de la constante diélectrique dans l'objet à examiner (1), $T(\underline{r})$ représente la répartition de la température dans l'objet à examiner (1), en fonction de laquelle l'équation intégrale doit être résolue, et G $(\underline{r}-\underline{r}')$ représente la fonction de Green.

4. Procédé d'hyperthermie, dans lequel une zone volumique prédéterminée de l'intérieur de l'objet à examiner (1) est réchauffée, la détermination de la répartition de la température dans l'objet à examiner (1) ayant lieu suivant un procédé tel qu'indiqué dans l'une des revendications 1 à 3.

5. Procédé d'hyperthermie selon la revendication 4, caractérisé par le fait que le réchauffage de la zone volumique prédéterminée à l'intérieur de l'objet à examiner (1), a lieu avant la détermination, sans contact, de la répartition de la température.

6. Procédé d'hyperthermie selon la revendication 4 ou 5, caractérisé par le fait que la zone volumique est chauffée à une température se situant environ entre 42 et 43°C.

7. Dispositif pour la mise en œuvre du procédé selon l'une des revendications 1 à 3, dans lequel l'objet à examiner (1) est disposé entre un émetteur hyperfréquence (3) et un premier dispositif-récepteur hypofréquence (5), du type dans lequel le premier dispositif de réception hypofréquence (5) est relié à un circuit d'évaluation (25, 27) qui détermine la répartition spatiale de la constante diélectrique dans l'objet à examiner (1) et l'applique à une première entrée d'une unité de calcul (29), dans lequel il est prévu, pour la saisie de la radiation thermique propre de l'objet à examiner (1), un second dispositif-récepteur hyperfréquence (31) qui applique les valeurs du rayonnement thermique propre, en valeur et en phase, à la seconde entrée de l'unité de calcul (29), et dans lequel, à la sortie de l'unité de calcul (29) on prélève un signal qui représente la répartition spatiale de la température dans l'objet à examiner (1).

8. Dispositif selon la revendication 7, caractérisé par le fait que le premier et le second dispositif-récepteur hyperfréquence (5, 31) sont accordés sur des fréquences différentes.

9. Dispositif selon la revendication 7 ou 8, caractérisé par le fait que le premier et le second dispositif récepteur hyperfréquence (5, 31) sont accordés à des fréquences respectives qui se situent entre 0,1 et 10 GHz.

10. Dispositif selon la revendication 7, caractérisé par le fait que le premier et le second dispositif récepteur hyperfréquence (5, 31) sont rassemblés en un dispositif récepteur hyperfréquence commun qui est relié aussi bien au circuit d'évaluation (25, 27) pour déterminer la répartition spatiale de la constante diélectrique qu'avec la seconde entrée de l'unité de calcul (29).

11. Dispositif selon l'une des revendications 7 à 10, caractérisé par le fait que le circuit d'évaluation (25, 27) pour déterminer la répartition spatiale de la constante diélectrique, comprend une mémoire (27) dans laquelle peut être introduite la constante diélectrique, et que la sortie de la mémoire (27) est reliée à l'unité de calcul (29).

12. Dispositif selon l'une des revendications 7 à 11, caractérisé par le fait qu'il comprend une source de chauffage (51) pour chauffer une zone volumique prédéterminée d'un objet à examiner (1).

13. Dispositif selon la revendication 12, caractérisé par le fait qu'il est prévu un dispositif de commande (49) qui débranche la source de chauffage (51) lorsque le maximum de la répartition de la température, déterminé par l'unité de calcul (29), dépasse une valeur limite prédéterminée.

14. Dispositif selon la revendication 13, caractérisé par le fait que la valeur limite prédéterminée est de l'ordre de 43°C.

15. Dispositif selon l'une des revendications 10 à 14, caractérisé par le fait qu'il est prévu une unité de commande (11) qui débranche l'émetteur hyperfréquence (3) lorsque le dispositif récepteur hyperfréquence commun (5) a saisi le rayonnement thermique propre de l'objet à examiner (1).

16. Dispositif selon l'une des revendications 11 à 15, caractérisé par le fait qu'il est prévu une unité de commande (11) qui branche l'émetteur hyperfréquence (3) lorsque la répartition spatiale de la constante diélectrique qui a été déterminée par le circuit d'évaluation (25, 27), doit être inscrite dans la mémoire (27).

17. Dispositif selon l'une des revendications 12 à 16, caractérisé par le fait que la source de chauffage (51) est reliée à un dispositif de commande (49) dont une entrée reçoit la répartition de la température et dont l'autre entrée (53) reçoit les coordonnées de la zone volumique prédéterminée dans l'objet à examiner (1), que dans le dispositif de commande (49) on détermine, à partir de la répartition de la température, les coordonnées du maximum de température, qui sont comparées avec les coordonnées prédéterminées, et que la direction du rayonnement de la source de chauffage (51) est ajustée automatiquement de manière que dans le cas d'un nouveau chauffage, les coordonnées du maximum de la répartition de la température coïncident avec les coordonnées prédéterminées.

18. Dispositif selon l'une des revendications 12 à 17, caractérisé par le fait que la source de chauffage (51) est une source d'irradiation à fréquence d'émission qui se situe en dehors de la largeur de bande de la fréquence du dispositif-récepteur (5, 31).

19. Dispositif selon l'une des revendications 10 à 18, caractérisé par le fait que la mesure active de la constante diélectrique et la mesure passive de l'émission thermique propre ont lieu de façon alternée.